# FASCICULE DE BREVET EUROPEEN

(11) **EP 2 555 771 B1**
(45) Date de publication et mention de la délivrance du brevet: **23.11.2016**
(21) Numéro de dépôt: 11714252.1
(22) Date de dépôt: 05.04.2011
(51) Int. Cl.: A61K 31/4188, A61P 27/02

(54) **COMPOSITIONS PHARMACEUTIQUES FORTEMENT DOSEES EN BIOTINE**
PHARMAZEUTISCHE ZUSAMMENSETZUNGEN MIT HÖHER DOSIERUNG IN BIOTINE
PHARMACEUTICAL COMPOSITIONS STRONGLY DOSED IN BIOTINE

(30) Priorité: 06.04.2010 FR 1052589
(43) Date de publication de la demande: 13.02.2013
(73) Titulaire: Assistance Publique - Hôpitaux de Paris, 75004 Paris (FR)
(72) Inventeur: SEDEL, Frédéric, F-75020 Paris (FR); BELLANGER, Agnès, F-75006 Paris (FR)
(74) Mandataire: Flesselles, Bruno F.G.
(86) Numéro de dépôt international: PCT/EP2011/055273
(87) Numéro de publication internationale: WO 2011/124571

(56) Documents cités:
- WO-A1-2004/017766
- US-A- 5 789 401
- WEBER PETER ET AL: "Outcome in patients with profound biotinidase deficiency: relevance of newborn screening", DEVELOPMENTAL MEDICINE & CHILD NEUROLOGY, vol. 46, no. 7, juillet 2004 (2004-07), pages 481-484, XP8126454, ISSN: 0012-1622
- RAHMAN SHAMIMA ET AL: "Late presentation of biotinidase deficiency with acute visual loss and gait disturbance", DEVELOPMENTAL MEDICINE AND CHILD NEUROLOGY, vol. 39, no. 12, décembre 1997 (1997-12), pages 830-831, XP8126455, ISSN: 0012-1622
- YANG YANLING ET AL: "Spinal cord demyelination associated with biotinidase deficiency in 3 Chinese patients", JOURNAL OF CHILD NEUROLOGY, vol. 22, no. 2, février 2007 (2007-02), pages 156-160, XP8126453, ISSN: 0883-0738
- PUERTAS BORDALLO D ET AL: "[Optic neuropathy in biotinidase deficiency]", ARCHIVOS DE LA SOCIEDAD ESPAÑOLA DE OFTALMOLOGÍA AUG 2004 LNKD- PUBMED:15306966, vol. 79, no. 8, août 2004 (2004-08), pages 393-396, XP8126451, ISSN: 0365-6691
- DATABASE WPI Week 199724 Thomson Scientific, London, GB; AN 1997-267712 XP002599161, & JP 9 095448 A (CALPIS SHOKUHIN KOGYO KK) 8 avril 1997 (1997-04-08)

## Description

L'invention se rapporte à de nouvelles compositions pharmaceutiques fortement dosées en biotine, ainsi qu'à leur utilisation dans le traitement de déficiences visuelles, notamment liées à des atrophies optiques.

La biotine (ou vitamine H) est une vitamine hydrosoluble ubiquitaire, naturellement retrouvée dans de nombreux aliments dont les abats, l'oeuf et certains légumes. La biotine apportée par l'alimentation provient de la biocytine (dimère biotine-lysine) produit de la dégradation des protéines par les protéases et peptidases digestives. La biotine libre est produite dans la lumière intestinale par action de la biotinidase. La biotine est ensuite transportée par plusieurs transporteurs dont le *sodium-dependent multivitamin transporter* (SMVT). L'élimination de la biotine est essentiellement urinaire sous forme de catabolites. Des carences acquises peuvent s'observer chez les sujets dénutris, ou en cas d'intoxication par l'avidine contenue dans le blanc d'oeuf (l'avidine se lie à la biotine dans la lumière du tube digestif et empêche son absorption).

Chez les mammifères, la biotine sert de co-facteur pour quatre carboxylases du métabolisme intervenant dans plusieurs étapes-clé du métabolisme énergétique dont la pyruvate carboxylase (néoglucogénèse), la 3-methylcrotonyl CoA et la propionyl CoA carboxylases (catabolisme de certains acides aminés qui fournissent des métabolites intermédiaires au cycle de Krebs) ; et l'acétyl CoA carboxylase (synthèse des acides gras).

Au cours des dernières années, il a en outre été montré que la biotine pouvait réguler l'expression de nombreux gènes via un mécanisme de biotinylation/dé-biotinylation des histones, édifices protéiques qui régulent la conformation de l'ADN et, par ce biais, l'accès de certaines régions du génome aux facteurs de transcription. Il semble qu'un grand nombre des gènes dont l'expression est régulée par la biotine codent pour de protéines impliquées dans le métabolisme énergétique (Zempleni et al., 2009).

Chez l'homme, deux maladies métaboliques héréditaires touchent directement le métabolisme de la biotine : le déficit en biotinidase et le déficit en holocarboxylase synthétase (Zempleni et al., 2009). La biotidinase est indispensable pour la libération de la biotine libre à partir de la biocytine générée par protéolyse. L'holocarboxylase synthase est, elle, impliquée dans la fixation de la biotine libre aux 4 apocarboxylases biotine dépendantes (3-methylcrotonyl CoA carboxylase, propionyl CoA carboxylase, pyruvate carboxylase et acétyl CoA carboxylase). Ces deux maladies ont des présentations cliniques polymorphes mais les premiers signes apparaissent en général dans la petite enfance: acidose métabolique, crises d'épilepsie, hypotonie, troubles de conscience, ataxie cérébelleuse, surdité de perception, rash cutané, alopécie. Ces symptômes peuvent être améliorés de façon spectaculaire (notamment en cas de déficit en biotinidase) par de petites doses de biotine (entre 5 et 20 mg/jour). Des formes tardives de déficit en biotinidase débutant à l'adolescence ou à l'âge adulte ont également été rapportées notamment chez quelques patients présentant des atteintes médullaires ou une atrophie optique (Ramaekers et al., 1993 ; Wolf et al., 1998). Dans certains cas, ces troubles neurologiques étaient réversibles sous biotine.

Par ailleurs, une maladie génétique, de transmission autosomique récessive, non directement liée au métabolisme de la biotine, et appelée « biotin-responsive basal ganglia disease » (BBGD, OMIM # 607483) a été décrit pour la première fois en 1998 chez 10 patients originaires du moyen orient (Ozand et al, 1998). Les patients atteints de BBGD présentaient des épisodes subaigus d'encéphalopathie souvent déclenchés par des épisodes fébriles et caractérisés par une confusion, une épilepsie, une ophtalmoplégie externe, une dysphagie, une faiblesse généralisée menant parfois au coma voire au décès. L'administration de très fortes doses de biotine (10 mg/kilo/jour) pendant ces accès permettait une récupération partielle ou complète en quelques jours. En absence de traitement, les patients présentaient des séquelles neurologiques graves, notamment une dystonie généralisée permanente en rapport avec des lésions bilatérales des noyaux gris centraux. Le gène responsable de ce syndrome a été identifié récemment: il s'agit de SLC19A3 codant pour la protéine hTHTR2 qui est un transporteur secondaire de la thiamine (Zeng et al, 2005). La biotine n'étant pas un substrat de hTHTR2 (Subramanian et al., 2006), le mécanisme précis par lequel la biotine améliore le phénotype clinique reste mystérieux.

Une hypothèse pourrait être que la biotine à très forte doses active l'expression du gène SLC19A3, permettant ainsi de restaurer la fonction du transporteur défectueux. Cette hypothèse de « thérapie d'activation transcriptionnelle » repose sur les observations suivantes
(1) la biotine régule de nombreux gènes (Zempleni, 2009), et
(2) l'expression du gène SLC19A3 diminue en cas de carence en biotine (Vlasova et al., 2005), indiquant que l'activité de ce gène est régulée (directement ou indirectement) par la biotine.

Une autre hypothèse serait que l'activation forcée par de fortes doses de biotine des enzymes biotine dépendantes conduise à une activation du cycle de Krebs via l'apport de substrats anaplérotiques générés par ces enzymes, qui pourrait pallier au déficit du transport de la thiamine, causé par les mutations de SLC19A3.

La biotine est présente en tant que principe actif dans un certain nombre de médicaments. Toutefois, ces composés contiennent une faible quantité de biotine par dose.

Dans la plupart des cas, ces médicaments contiennent également d'autres principes actifs (notamment d'autres vitamines). Ces produits sont utilisables par voie orale. Ces produits ayant plusieurs principes actifs contiennent moins de 1 mg de biotine (entre 0,15 et 0,2 mg) par dose unitaire (tablette ou comprimé).

Un médicament vendu en France (Biotine Bayer@) se présente sous forme de comprimés ou de solution injectable et ne contient que de la biotine, en tant que principe actif, ainsi que des excipients. Ce médicament est utilisé comme traitement d'appoint des alopécies diffuses. La biotine Bayer contient 5 mg de biotine par dose unitaire (comprimé ou ampoule injectable).

Dans le cadre de la présente invention, une nouvelle forme pharmaceutique (nouveau dosage) de biotine a été développée, permettant une administration d'une grande quantité de biotine par voie orale pour chaque dose unitaire. Ces médicaments peuvent être utilisés pour le traitement de maladies induisant des déficiences visuelles par neuropathie optique, et notamment certaines formes de leucoencéphalopathies présentant des signes cliniques originaux.

Les fortes doses de biotine administrées par les compositions selon l'invention permettent une amélioration des signes cliniques, dont on fait l'hypothèse qu'elle est liée à une bonne assimilation de la biotine apportée. Il est à noter qu'aucun effet toxique de la biotine à très fortes doses n'a été décrit dans la littérature médicale ou chez les patients traités par les inventeurs.

Dans un premier aspect, l'invention se rapporte à une composition pour une administration par voie orale, contenant 100 mg de biotine. Cette composition est à usage pharmaceutique, et est donc un médicament. Il est entendu que chaque dose unitaire de la composition selon l'invention contient 100 mg de biotine, en tant que principe actif.

Dans un mode de réalisation particulier, la composition selon l'invention contient de la biotine en tant que principe actif unique, ainsi que des excipients, sans autre principe actif.

Un excipient doit s'entendre comme tout composé formant partie de la formulation qui est destiné à agir comme simple support, c'est-à-dire qui n'est pas destiné à avoir une activité biologique.

La composition selon l'invention peut se présenter sous toute forme connue dans l'art. En particulier, elle se présente sous la forme de gélules, de comprimés (pelliculés ou non), de pilules ou de tablettes. Dans un autre mode de réalisation, elle se présente sous la forme d'un sirop. Ledit sirop est dosé de telle sorte qu'il contienne 100 mg de biotine par dose unitaire. La concentration de biotine dans ce sirop est fonction de la dose unitaire que l'on souhaite donner au patient.

Des excipients utilisables par l'homme du métier sont bien connus dans l'art. On peut ainsi choisir le talc (E553b), la cellulose microcristalline, le lactose, l'amidon (en particulier de maïs), le stéarate de magnésium (E572), l'acide stéarique (E570).

Lorsque que l'on prépare la composition selon l'invention sous forme de gélules, un excipient préféré est la cellulose microcristalline.

Lorsque la composition se présente sous la forme d'un comprimé pelliculé, ladite pellicule peut être formée de toute substance connue dans l'art, telle que l'hypromellose (E464), l'éthylcellulose, la macrogol, le Talc (E553b), le dioxyde de titane (E171), l'oxyde de fer (E172).

On peut également colorer le principe actif (par tout colorant acceptable, tel que le carmin), ce qui permet de vérifier la bonne dispersion de la biotine dans l'excipient.

La demande décrit également une composition injectable contenant au moins 20 mg, de préférence au moins 40 mg de biotine par dose unitaire, de préférence au moins 50 mg, de façon plus préférée au moins 75 mg, de façon la plus préférée au moins 100 mg de biotine par dose unitaire.

Cette composition injectable peut se présenter sous la forme d'une ampoule contenant la biotine, ainsi que des excipients acceptables. On ajuste la concentration de biotine selon le volume envisagé de l'ampoule. Certains excipients améliorant la solubilité de la biotine peuvent être utilisés.

Les excipients utilisables pour la fabrication de compositions injectables sont bien connus dans l'art. On peut notamment citer le dihydrogénophosphate de sodium, le bicarbonate de sodium (E550i), le parahydroxybenzoate de méthyle (E218), le parahydroxybenzoate de propyle (E216), qui peuvent être utilisés ensemble dans des proportions que sait déterminer l'homme du métier. L'eau utilisée est destinée aux préparations injectables. L'injection se fait de préférence par voie intramusculaire. Elle peut également être réalisée par voie intraveineuse.

Les compositions fortement dosées en biotine sont particulièrement intéressantes et adaptées pour une utilisation dans le traitement d'une déficience ou d'une atrophie visuelle. Ce traitement peut être un traitement principal ou un traitement d'appoint d'un traitement principal visant à s'attaquer aux causes de l'atrophie visuelle. L'invention se rapporte également à l'utilisation de biotine pour la préparation d'un médicament destiné à traiter une déficience ou une atrophie visuelle (ou tout autre pathologie citée plus bas), ainsi qu'aux méthodes de traitement de ces pathologies par administration de biotine.

Les compositions fortement dosées en biotine sont particulièrement intéressantes et adaptées pour stabiliser l'état clinique d'un patient atteint d'une déficience ou d'une atrophie visuelle.

Pour une telle utilisation, la quantité de biotine administrée au patient doit être au moins égale à 3 mg/kg/jour, de façon plus préférée 5 mg/kg/jour, ou au moins égale à 7,5 mg/kg/jour, voire autour de 10 mg/kg/jour. On administre entre 100 et 700 mg de biotine par jour aux patients, généralement entre 200 et 500 mg par jour, généralement autour de 300 mg par jour.

L'atrophie visuelle (ou atrophie optique) est généralement due à une atrophie du nerf optique, s'accompagnant d'une modification du champ visuel et d'une baisse de l'acuité visuelle. Elle peut être provoquée par un processus inflammatoire, tumoral, vasculaire ou toxique.

Dans un mode de réalisation particulier, l'atrophie visuelle est observée en l'absence d'une étiologie claire telle que mentionnée ci-dessus.

Dans ce mode de réalisation, l'atrophie visuelle est un symptôme lié à une leuco-encéphalopathie particulière, c'est-à-dire une atteinte de la substance blanche du cerveau.

Cette leuco-encéphalopathie peut être caractérisée par les éléments suivants : atteinte des régions suivantes de la substance blanche cérébrale : substance blanche péri-ventriculaire, radiations optiques, faisceaux cortico-spinaux, pédoncules cérébelleux, ainsi que l'on peut l'observer par IRM cérébrale, et une élévation du pic de choline dans le centre semi-ovale, ainsi qu'il peut être observé par spectroscopie par résonance magnétique nucléaire (SRMN).

Ainsi, on observe des anomalies IRM spécifiques et non retrouvées au cours d'autres leucoencéphalopathies d'origine métabolique (revue dans Sedel et al., 2008) : (1) hypersignal des faisceaux cortico-spinaux ; (2) hypersignal des radiations optiques ; (3) hypersignal des pédoncules cérébelleux ; (4) hypersignal modéré de la substance blanche péri-ventriculaire.

Sur le plan électrophysiologique, les patients présentent des anomalies évoquant une atteint uni-ou bi-latérale des nerfs optiques, même en absence de baisse d'acuité visuelle.

Une réaction inflammatoire (hypercellularité >4 éléments/mm³) est également souvent observée chez les patients.

Cette leuco-encéphalopathie sensible à de fortes doses de biotine s'accompagne ainsi d'une atteinte uni-ou bilatérale des nerfs optiques qui peut être symptomatique (baisse d'acuité visuelle) ou infra-clinique (détectée uniquement sur les potentiels évoqués visuels, sans signe clinque), qui s'améliore sous traitement. Le syndrome cérébelleux et l'élévation du pic de choline observée par SRMN s'améliorent sous traitement.

Le diagnostic de cette « leuco-encéphalopathie biotine sensible » repose sur des critères cliniques, radiologiques et neurophysiologiques :
a) critères cliniques : maladie évoluant par poussées s'installant sur quelques jours et au cours desquels le patient peut présenter les symptômes suivants ; syndrome cérébelleux, baisse de l'acuité visuelle unilatérale. Entre ces épisodes subaigus, les patients peuvent présenter des céphalées, des troubles psychiatriques, et des séquelles des poussées antérieures : syndrome cérébelleux permanent, baisse de l'acuité visuelle uni ou bi latérale permanente.
b) critères radiologiques : L'IRM cérébrale montre une leuco-encéphalopathie caractéristique qui intéresse les régions suivantes de la substance blanche cérébrale : substance blanche péri-ventriculaire, radiations optiques, faisceaux cortico-spinaux, pédoncules cérébelleux. L'IRM des nerfs optiques peut montrer une atrophie optique uni- ou bi-latérale. La spectroscopie par résonance magnétique nucléaire (SRMN) montre une élévation du pic de choline dans le centre semi-ovale.
c) critères neurophysiologiques : l'étude des potentiels évoqués visuels peut montrer une augmentation des ondes P100 bilatérale en rapport avec une atteinte des deux nerfs optiques ou une absence d'onde P100 dans les cas sévères. Le potentiel évoqué visuel (PEV) est la réponse électrique du cortex qui est provoquée par une stimulation visuelle. Les PEV résultent de l'enregistrement des variations de potentiels générés par l'activité bio-ionique du cortex occipital consécutif à un stimulus visuel dont un paramètre varie dans le temps. Les PEV étudient le fonctionnement maculaire et périmaculaire ainsi que la conduction des voies visuelles.

On administre la composition selon l'invention aux patients présentant les critères sus-définis, à une dose telle que mentionnée ci-dessus, en particulier, 100 mg trois fois par jours pendant 3 mois.

En cas d'amélioration certaine d'un des paramètres après réévaluation clinique, radiologique (IRM+SRMN) et électrophysiologique, le traitement peut être poursuivi. La dose de biotine peut être adaptée (augmentée ou diminuée).

Si aucun des paramètres ne s'est amélioré de façon certaine, mais que l'on a une forte suspicion diagnostique (clinique, IRM, potentiels évoqués visuels fortement évocateurs), on poursuit le traitement thérapeutique pendant 3 mois supplémentaires (en adaptant éventuellement la dose de biotine) au terme desquels une nouvelle évaluation clinique, radiologique (IRM+SRMN) et électrophysiologique est effectuée.

On peut également utiliser les compositions selon l'invention pour le traitement de patients souffrant d'autres pathologies :
a) suspicion de BBGD : épisodes d'encéphalopathie déclenchées par des épisodes fébriles et au cours desquels l'IRM cérébrale montre des lésions en hypersignal FLAIR/T2 des noyaux gris centraux (putamen et noyaux caudés). Une posologie de 10mg/kilo/jour doit être proposée durant les épisodes d'encéphalopathie, associée à de la vitamine B1 (500 mg/jour) (Debs et al., 2010). Ce test thérapeutique doit être accompagné d'une étude génétique avec recherche de mutations dans le gène SLC19A3.
b) suspicion de pathologie biotine sensible : affection neurologique « sans diagnostic » évoluant par épisodes subaigus et associant diversement les signes cliniques suivants : atrophie optique uni-ou bi-latérale, syndrome cérébelleux subaigu, atteinte des noyaux gris centraux.
c) autre pathologie neurologique potentiellement liée à un trouble du métabolisme énergétique : on peut citer la maladie d'Alzheimer, la chorée de Huntington, la maladie de Parkinson, ou certaines épilepsies symptomatiques. La biotine fortement dosée peut ainsi être utilisée comme traitement d'appoint pour réduire les symptômes observés pour ces maladies.

### Description des figures

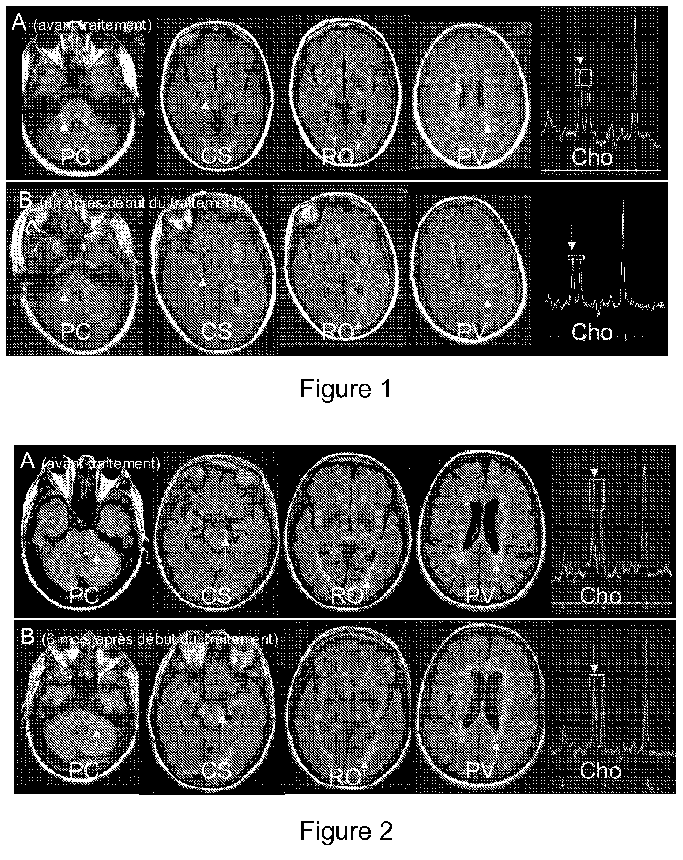

Figure 1 : Contrôles IRM et spectroscopie RMN réalisés sur la patiente 1 avant (figure 1A) et après traitement (figure 1B). Sur l'IRM réalisée avant traitement, on observe une leucoencéphalopathie touchant les pédoncules cérébelleux (PC), les faisceaux cortico-spinaux (CS), les radiations optiques (RO), et la substance blanche péri-ventriculaire (PV). La spectroscopie par RMN montre une augmentation du pic de choline (Cho, pic le plus à gauche), qui devrait être à une hauteur semblable au pic situé juste à sa droite. Après traitement, les signes de leucoencéphalopathie et le pic de choline ont nettement diminué.
Figure 2 : Contrôles IRM et spectroscopie RMN réalisés sur la patiente 2 avant (figure 2A) et après traitement (figure 2B). Sur l'IRM réalisée avant traitement, on observe une leucoencéphalopathie touchant les pédoncules cérébelleux (PC), les faisceaux cortico-spinaux (CS), les radiations optiques (RO), et la substance blanche péri-ventriculaire (PV). La spectroscopie par RMN montre une augmentation du pic de choline (Cho). Après traitement, le pic de choline a diminué.

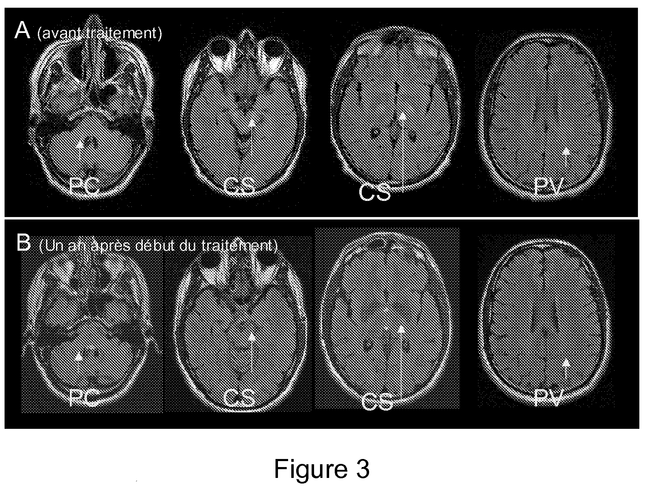

Figure 3: Contrôles IRM réalisés sur le patient 3 avant (figure 3A) et après traitement (figure 3B). Sur l'IRM réalisée avant traitement, on observe une leucoencéphalopathie touchant les pédoncules cérébelleux (PC), les faisceaux cortico-spinaux (CS), les radiations optiques (RO), et plus faiblement la substance blanche péri-ventriculaire (PV). Après traitement, les signes de leuco-encéphalopathie ont disparu.

Les exemples suivants montrent l'intérêt d'un traitement avec les préparations selon l'invention.

### Exemples

### Exemple préliminaire : Préparation de gélules fortement dosée en biotine

La biotine, matière première, est obtenue auprès d'un grossiste établissement pharmaceutique LA COOPER (Coopération Pharmaceutique Française) de MELUN. Avant mélange avec l'excipient (cellulose microcristalline), une pincée de carmin est ajoutée au principe actif (traceur de la bonne répartition dans le mélange). La répartition du mélange est ensuite réalisée dans des gélules n°1 (0,50 ml).

Pour 100 gélules dosées à 100 mg, on effectue le mélange suivant :
- biotine : 10 g
- carmin : une pincée
- cellulose microcristalline : qsp 50 ml

### Exemple 1

Un patient de sexe féminin (54 ans en 2010) a présenté une baisse de l'acuité visuelle de l'oeil gauche suivie d'une baisse d'acuité visuelle de l'oeil droit, 10 jours plus tard (mai 2002). La baisse d'acuité visuelle est indolore mais a été précédée de cervicalgies et de céphalées. Une atrophie optique s'est installée rapidement. En décembre 2002, elle présente sur plusieurs jours des troubles de l'équilibre, des troubles sphinctériens (dysurie) et des paresthésies des 4 membres.

L'examen neurologique met en évidence des Réflexes vifs aux 4 membres avec signe de Babinski bilatéral, ainsi qu'un syndrome cérébelleux cinétique du membre inférieur Gauche.

L'IRM réalisée à cette époque montre une leucoencéphalopathie touchant la substance blanche périventriculaire, les faisceaux cortico-spinaux au niveau des capsules internes et du tronc cérébral avec un hypersignal franc, les radiations optiques et les pédoncules cérébelleux, surtout à droite. La spectro RMN du 6 décembre 2002 ne montre pas d'élévation du pic de choline. L'IRM médullaire est normale. Plusieurs contrôles IRM en juillet 2003 et mai 2004 montrent un aspect superposable (figure 1A). Le pic de choline est cependant élevé de façon anormale sur la spectro RMN de mai 2004.

En juillet 2003, elle compte les doigts à 20cm à droite et voit la main bouger à gauche.

En mai 2004, l'acuité visuelle est chiffrée à 1,6/10 à Droite et à 1/50 ème à gauche. Cette acuité visuelle reste stable jusqu'en avril 2006 où un traitement par la biotine à la dose de 20 mg/jour est introduit.

Après deux mois de traitement, la patient a l'impression de voir un peu mieux. Après trois mois de traitement (07/2006), l'acuité visuelle est chiffrée à 4/10 à droite et toujours 1/50 à gauche. Cette amélioration initiale se maintient au contrôle à 7 mois (Nov 2006).

En décembre 2006, le traitement est augmenté à 100 mg/jour.

Après deux semaines, la patiente commence à pouvoir lire les gros titres d'un journal.

En juillet 2007 (après 15 mois de traitement), l'acuité visuelle est notée à 6/10 à droite et à 1/20 à gauche. Les PEVs visuels montrent alors une réponse corticale à droite retardée à +4,5DS (la réponse était nulle avant traitement).

La patiente signale également une amélioration de ses problèmes d'équilibre. L'IRM du mois de Juillet (2007) montre une normalisation du pic de choline, ainsi qu'une diminution nette de l'intensité de signal de la leucoencéphalopathie (figure 1 B).

### Exemple 2

Un traitement à base de biotine fortement dosé a été donné à une patiente de 72 ans (en 2010). Cette patiente se plaignait de migraines depuis l'âge de 20 ans.

En 2004 (66 ans), elle présenta des troubles psychiatriques à type de délire de persécution pendant un an. Une psychose maniaco-dépressive a été diagnostiquée, à la vue de la récurrence des troubles à type d'accès maniaques (2 ou 3 épisodes en tout).

En 2006, 2007 et 2008, elle présenta des troubles de la marche paroxystiques décrits comme des troubles de l'équilibre associés à une faiblesse des membres inférieurs qui duraient à chaque fois, moins de 24 heures.

Le 1er épisode de 2006 a été accompagné d'une fièvre et la ponction lombaire de l'époque montrait 17 éléments (lymphocytes), une protéinorachie à 0,3 g/l. Entre ces épisodes, elle conservait des troubles de l'équilibre discrets.

Fin 2006 est apparue une baisse de l'acuité visuelle bilatérale indolore, prédominant du côté droit, d'aggravation par paliers » entre 2006 et 2007 (en 2007, elle comptait seulement les doigts à droite et l'acuité visuelle à gauche était évaluée à 2/10ème).

Au cours de l'été 2008, les troubles de l'équilibre devinrent permanents.

L'examen clinique en mai 2009 montrait un discret syndrome cérébelleux statique, mais également cinétique du membre supérieur droit. Les réflexes ostéo tendineux étaient vifs aux quatre membres, mais il n'existait pas de signe de Babinski.

L'IRM cérébrale montrait une leucoencéphalopathie faite d'hyper signaux péri-ventriculaires, des faisceaux pyramidaux au niveau des pédoncules cérébraux et du diencéphale. Cette leucoencéphalopathie touchait aussi les radiations optiques et dans une moindre mesure les pédoncules cérébelleux (Figure 2A).

Les potentiels évoqués visuels réalisés en juin 2009 ne montraient aucune réponse corticale. L'acuité visuelle était chiffrée à 1 à droite ; 2/10 à gauche. A l'époque, la patiente se plaignait en outre de céphalées fréquentes (au moins une crise par semaine). Un traitement par biotine (3 x 100 mg/jour) fut débuté en juin 2009.

En Septembre 2009, la patiente notait une amélioration de son acuité visuelle : elle pouvait lire les numéros de téléphone, elle distinguait les visages et pouvait lire les gros titres des journaux. Elle signalait une diminution importante de la fréquence des céphalées : 1 à 2/mois. L'équilibre était plus assuré, notamment lors du demi-tour. Elle pouvait faire la cuisine seule ce qui n'était pas le cas auparavant. L'IRM était inchangée de même que la spectro IRM cérébrale. Par contre les potentiels évoqués visuels montraient la réapparition d'une onde P100 à gauche (aucune réponse n'était notée à droite) de latence allongée (126,5 ms).

Le traitement fut poursuivi à la même dose. En Janvier 2010 (après 6 mois de traitement), les potentiels évoqués montraient une ébauche d'onde P100 à droite ainsi qu'une amélioration de la latence de l'onde P100 gauche (passée de 126,5 à 111,8 ms). La spectro IRM cérébrale montrait une nette diminution du pic de choline et du rapport choline/créatine alors que la leucoencéhalopathie demeurait inchangée (Figure 2B). Le traitement par biotine a été augmenté à 600 mg/jour. Ce traitement est toujours en cours.

### Exemple 3

Un patient de 40 ans (né en 1970) présentait plusieurs épisodes de diplopie transitoires pour lesquels il avait consulté en 2004, sans qu'il soit possible de retrouver une étiologie.

En Janvier 2007, il présenta le matin au réveil une dysarthrie pseudo-ébrieuse. Cette dysarthrie s'aggrava légèrement entre Janvier et Février 2007. En mars 2007, après hospitalisation, l'examen clinique montrait simplement une dysarthrie ataxique ainsi qu'un léger syndrome cérébelleux statique. L'examen clinique était normal par ailleurs. La ponction lombaire montrait un liquide inflammatoire avec 7 éléments/mm³ (lymphocytes). L'IRM cérébrale montrait une leucoencéphalopathie touchant les faisceaux cortico-spinaux au niveau des capsules internes et du tronc cérébral, les radiations optiques et plus faiblement la substance blanche péri-ventriculaire (Figure 3A). L'acuité visuelle était normale.

En mars 2007, un traitement par biotine (200 mg/jour) fut débuté pour une durée totale de 2 mois. Bien qu'aucune amélioration clinique ne fût constatée, l'IRM de contrôle réalisée en Janvier 2008 montrait une disparition complète de la leuco-encéphalopathie (Figure 3B). Les potentiels évoqués visuels montraient des composantes corticales de latence et morphologie normale à droite mais d'amplitude réduite. A gauche, la réponse corticale était dispersée. Ces résultats étaient en faveur d'un dysfonctionnement infra-clinique des voies visuelles gauches.

Ce patient a été perdu de vue d'Octobre 2008 à Juillet 2010 et a arrêté son traitement pendant ce laps de temps. Son état neurologique s'est aggravé notamment sa dysarthrie et ses troubles de l'équilibre et cette aggravation a conduit à l'utilisation d'un fauteuil roulant. Durant ce laps de temps, une atrophie du tronc cérébral et du cervelet est apparue sur l'IRM cérébrale. La reprise du traitement a permis la stabilisation de l'état de ce patient.

### Exemple 4

Une patiente de 49 ans, née en 1961, présentait des antécédents d'uvéite postérieure bilatérale compliquée d'une choriorétinite et d'une cataracte bilatérale. Aucune étiologie n'a été retrouvée malgré un bilan très complet. Elle présente un surpoids et un diabète de type 2. Elle est suivie en psychiatrie depuis 1998 pour des troubles psychotiques apparus suite à l'accouchement de son fils. Depuis cette époque, elle a été hospitalisée de façon itérative pour des épisodes délirants avec hallucinations oniroïdes. Une IRM a été réalisée dans le cadre du bilan de sa maladie et montre une leucoencéphalopathie touchant les faisceaux cortico-spinaux, les pédoncules cérébelleux, les radiations optiques et la substance blanche périventriculaire. L'examen clinique montrait une ataxie cérébelleuse très discrète mais rien de plus.

En Septembre 2008, un traitement par biotine (100 mg deux fois par jour) fut débuté. Trois mois plus tard (Décembre), l'IRM de contrôle montrait une diminution significative du pic de choline mais sans modification de la leucoencéphalopathie. La patiente fut ensuite perdue de vue.

### Conclusion

Le tableau I suivant résume les symptômes cliniques et observations radiologiques et neurophysiologiques chez les patients des exemples 1 à 4.

**Tableau I : caractéristiques avant traitement**

| Patient | 1 | 2 | 3 | 4 |
|---|---|---|---|---|
| **Caractéristiques cliniques** | | | | |
| Sexe | F | F | M | F |
| Age dernier examen | 53 | 71 | 38 | 47 |
| Age de début des troubles | 46 | 68 | 34 | 37 |
| Baisse acuité visuelle bilatérale | + | + | - | + |
| Syndrome cérébelleux | + | + | + | + |
| Syndrome pyramidal | + | - | - | - |
| Troubles psychiatriques | - | + | - | + |
| céphalées | + | + | - | - |
| **Caractéristiques radiologiques**, **électrophysiologiques et biologiques** | | | | |
| Leuco-encéphalopathie péri-ventriculaire | + | + | - | + |
| Hypersignal des radiations optiques | + | + | + | + |
| Hypersignal des pédoncules cérébelleux | + | + | - | + |
| Hypersignal des faisceaux cortico-spinaux | + | + | + | + |
| Élévation de la choline (SRMN) | + | + | NF | + |
| PEV: retard des ondes 100 ou absence de réponse | + | + | + | NF |
| EMG | NI | NI | NI | NI |
| Éléments par mm³ dans le liquide céphalo-rachidien (LCR) | 8 | 17 | 7 | 3 |
| Protéines dans le LCR | 0,72 | 0,3 | 0,5 | 0,34 |

| | | | | |
|---|---|---|---|---|
| NF : non fait | | | | |

Ainsi, tous les patients ont présenté une amélioration clinique ou para-clinique suivant l'introduction d'un traitement par la biotine à fortes doses.

Cette amélioration a porté sur les manifestations cliniques (acuité visuelle et ataxie) chez 2 patients / 4, (patients 1 et 2) sur les anomalies d'IRM (diminution de l'hypersignal de la substance blanche) chez 2 patients / 4 (patients 1 et 3), sur les anomalies de spectroscopie par résonance magnétique (diminution du pic de choline dans le centre semi-ovale) chez 3 patients / 3 (1, 2, 4) et sur les potentiels évoqués visuels chez 2 patients / 2 (patients 1 et 2). Il faut noter que les deux patients n'ayant pas éprouvé d'amélioration clinique nette sous traitement (alors que les paramètres IRM ou de spectroscopie étaient améliorés) ne présentaient que très peu de symptômes avant début du traitement.

Tous les patients n'ont pas eu la même durée de suivi sous traitement (entre 3 mois et 1 an). Il semble néanmoins que les premiers signes à s'améliorer soient :
1) la baisse de l'acuité visuelle chez les patients présentant une diminution avant traitement (patients 1 et 2);
2) la baisse du pic de choline évaluée en spectro RMN et l'amélioration des potentiels évoqués visuels puis
3) après au moins un an, la diminution de l'hypersignal de la substance blanche en IRM (observée chez les 2 patients suivis en IRM pendant un an après traitement (patients 1 et 3).

**Tableau II : résultats après traitement**

| Patient | Amélioration acuité visuelle | Amélioration syndrôme cérébelleux | Amélioration leucopathie (IRM) | Amélioration pic de choline | Amélioration des PEVs |
|---|---|---|---|---|---|
| 1 | + | + | + | + | + |
| 2 | + | +/- | - | + | + |
| 3 | - | - | + | NF | NF |
| 4 | - | - | - | + | NF |

| | | | | | |
|---|---|---|---|---|---|
| NF : non fait | | | | | |

### Références

Dabbagh O, Brismar J, Gascon GG, Ozand PT. The clinical spectrum of biotin-treatable encephalopathies in Saudi Arabia. Brain Dev. 1994;16 Suppl:72-80.
Debs R, Depienne C, Rastetter A, Bellanger A, Degos B, Galanaud D, Keren B, Lyon-Caen O, Brice A, Sedel F. Biotin-Responsive Basal Ganglia Disease (BBGD) in Europeans with novel SLC19A3 mutations. Arch Neurol. 2010 Jan;67(1):126-30.
Ozand PT, Gascon GG, Al Essa M, Joshi S, Al Jishi E, Bakheet S, Al Watban J, AI-Kawi MZ, Dabbagh O. Biotin-responsive basal ganglia disease: a novel entity.
Brain. 1998 Jul;121 (Pt 7):1267-79.
Ramaekers VT, Brab M, Rau G, Heimann G. (1993) Recovery from neurological deficits following biotin treatment in a biotinidase Km variant. Neuropediatrics 24:98-102.
Sedel F, Tourbah A, Fontaine B, Lubetzki C, Baumann N, Saudubray JM, Lyon-Caen O. Leukoencephalopathies associated with Inborn Errors of Metabolism in adults: a diagnostic approach. J Inherit Metab Dis. 2008 Jun;31(3):295-307.
Subramanian VS, Marchant JS, Said HM. Biotin-responsive basal ganglia disease-linked mutations inhibit thiamine transport via hTHTR2 : biotin is not a substrate for hTHTR2. Am J Physiol. 2006;291(5):851-859
Vlasova TI, Stratton SL, Wells AM, Mock NI, Mock DM. Biotin deficiency reduces expression of SLC19A3, a potential biotin transporter, in leukocytes from human blood. J Nutr. 2005;135(1): 42-47.
Wolf B, Pomponio RJ, Norrgard KJ, Lott IT, Baumgartner ER, Suormala T, Ramaekers VT, Coskun T, Tokatli A, Ozalp I, Hymes J. J Pediatr. 1998;132(2):362-5.
Zempleni J, Wijeratne SS, Hassan YI. Biotin. Biofactors 2009;35(1):36-46.
Zeng WQ, Al-Yamani E, Acierno JS Jr et al. Biotin-responsive basal ganglia diease maps to 2q36.3 and is due to mutations in SCL19A3. Am J Hum Genet. 2005;77(1):16-26.

## Revendications

1. Composition pharmaceutique pour une administration par voie orale, contenant 100 mg de biotine par dose unitaire.

2. Composition selon la revendication 1, **caractérisé en ce qu'**elle se présente sous la forme de gélules, de comprimés (pelliculés ou non), de tablettes ou de pilules.

3. Composition selon la revendication 1 ou 2, **caractérisé en ce qu'**elle contient de la biotine et des excipients, sans autre principe actif.

4. Composition selon l'une des revendications 1 à 3, **caractérisé en ce qu'**elle contient des excipients choisis parmi le talc, la cellulose microcristalline, le lactose, l'amidon, le stéarate de magnésium et l'acide stéarique.

5. Biotine pour son utilisation dans le traitement d'une déficience visuelle, dans laquelle ladite déficience visuelle est liée à une leucoencéphalopathie intéressant les régions suivantes de la substance blanche cérébrale : substance blanche péri-ventriculaire, radiations optiques, faisceaux cortico-spinaux, pédoncules cérébelleux, ainsi qu'observé par IRM cérébrale, et une élévation du pic de choline dans le centre semi-ovale, ainsi qu'observé par spectroscopie par résonance magnétique nucléaire, dans laquelle la quantité de biotine administrée au patient est au moins égale à 3 mg/kg/jour.

6. Biotine pour son utilisation dans le traitement d'une déficience visuelle selon la revendication 5, dans laquelle la quantité de biotine administrée au patient est au moins égale à 5 mg/kg/jour.

## Patentansprüche

1. Pharmazeutische Zusammensetzung für die orale Verabreichung, die 100 mg Biotin pro Dosiseinheit enthält.

2. Zusammensetzung nach Anspruch 1, **dadurch gekennzeichnet, dass** sie in Form von Kapseln, (überzogenen oder nicht überzogenen) Tabletten, Pastillen oder Pillen dargereicht wird.

3. Zusammensetzung nach Anspruch 1 oder 2, **dadurch gekennzeichnet, dass** sie Biotin und Exzipienten ohne anderen Wirkstoff enthält.

4. Zusammensetzung nach einem der Ansprüche 1 bis 3, **dadurch gekennzeichnet dass** sie Exzipienten enthält, die aus Talk, mikrokristalliner Cellulose, Lactose, Stärke, Magnesiumstearat und Stearinsäure ausgewählt sind.

5. Biotin für die Verwendung zur Behandlung einer Sehbehinderung, wobei die Sehbehinderung mit einer Leukoenzephalopathie einhergeht, die die folgenden Regionen der weißen Substanz des Gehirns betrifft: periventrikuläre weiße Substanz, Sehstrahlungen, kortikospinale Bahnen, Kleinhirnstiele, wie mittels Gehirn-MRT beobachtet, und eine Erhöhung des Cholin-Maximums im Centrum semiovale, wie mittels magnetischer Kernresonanzspektroskopie beobachtet, wobei die Menge an Biotin, die dem Patienten verabreicht wird, mindestens gleich 3 mg/kg/Tag ist.

6. Biotin für die Verwendung zur Behandlung einer Sehbehinderung nach Anspruch 5, wobei die Menge an Biotin, die dem Patienten verabreicht wird, mindestens gleich 5 mg/kg/Tag ist.

## Claims

1. Pharmaceutical composition for oral administration, containing 100 mg of biotin per unit dose.

2. Composition according to Claim 1, **characterized in that** it is in the form of gel capsules, tablets (optionally film-coated), lozenges or pills.

3. Composition according to Claim 1 or 2, **characterized in that** it contains biotin and excipients, without any other active ingredient.

4. Composition according to one of Claims 1 to 3, **characterized in that** it contains excipients chosen from talc, microcrystalline cellulose, lactose, starch, magnesium stearate and stearic acid.

5. Biotin for use thereof in the treatment of a visual impairment, in which the visual impairment is related to a leukoencephalopathy involving the following regions of the white matter of the brain: periventricular white matter, optic radiations, corticospinal tracts, cerebellar peduncles, as observed by brain MRI, and an elevation of the choline peak in the centrum semiovale, as observed by nuclear magnetic resonance spectroscopy, in which the amount of biotin administered to the patient is at least equal to 3 mg/kg/day.

6. Biotin for use thereof in the treatment of a visual impairment according to Claim 5, in which the amount of biotin administered to the patient is at least equal to 5 mg/kg/day.
